# EUROPÄISCHE PATENTSCHRIFT

(11) **EP 3 095 398 B1**
(45) Veröffentlichungstag und Bekanntmachung des Hinweises auf die Patenterteilung: **07.06.2023**
(21) Anmeldenummer: 16170232.9
(22) Anmeldetag: 18.05.2016
(51) Int. Cl.: A61B 17/15, A61B 17/56

(54) **PATIENTENSPEZIFISCHES INSTRUMENT ZUR REFERENZIERUNG VON KÖRPERTEILEN**
PATIENT-SPECIFIC INSTRUMENT FOR THE REFERENCING OF BODY PARTS
INSTRUMENT SPÉCIFIQUE AU PATIENT POUR RÉFÉRENCER DES PARTIES DU CORPS

(30) Priorität: 22.05.2015 CH 7242015
(43) Veröffentlichungstag der Anmeldung: 23.11.2016
(73) Patentinhaber: Medivation AG, 4200 Brugg (CH)
(72) Erfinder: SCHWÄGLI, Tobias, 5400 Solothurn (CH); STIFTER, Jan, 5425 Schneisingen (CH)
(74) Vertreter: Herrmann, Johanna

(56) Entgegenhaltungen:
- WO-A1-2012/152900
- WO-A1-2014/026083
- WO-A1-2014/140808
- US-A1- 2014 018 813

## Beschreibung

Die Erfindung betrifft ein patientenspezifisches Instrument zur Referenzierung von Körperteilen, insbesondere Knochenstrukturen. Mittels präoperativer Planung basierend auf CT- oder MRI-Daten des Patienten werden von der zu operierenden Knochenstruktur genaue 3D- Modelle erstellt. Diese 3D-Modelle werden nachfolgend als Knochenmodelle bezeichnet. Passend zu diesen Knochenmodellen können mittels generativer Verfahren patientenspezifische Instrumente gefertigt werden, welche sich eindeutig auf die Knochenstruktur aufsetzen lassen und somit eine Referenz zur präoperativen Bildgebung, respektive Planung, ermöglichen.

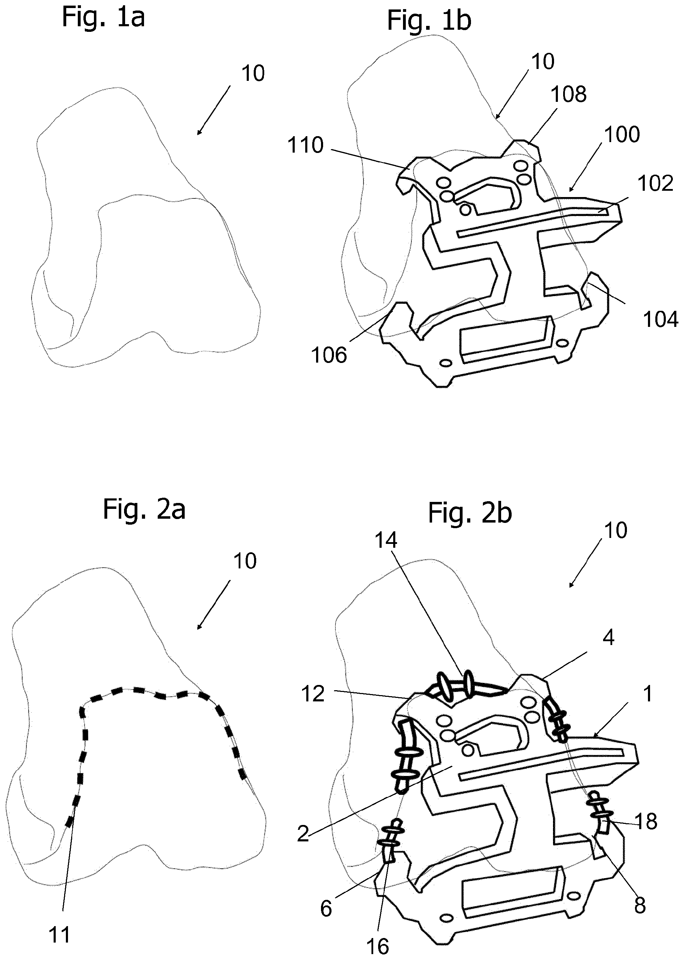

Eine vorbekannte Ausprägung solcher patientenspezifischer Instrumente sind patientenspezifische Schnittblöcke für den Kniegelenksersatz (siehe Fig. 1). Anhand der präoperativen Bilddaten werden die Implantatposition und damit die erforderlichen Knochenschnitte an Femur und Tibiaknochen definiert. Die patientenspezifischen Instrumente ermöglichen einen effizienteren und einfacheren OP-Ablauf verglichen mit konventionellen Instrumenten, da das Aufsetzverhalten und die Aufsetzgenauigkeit der patientenspezifischen Instrumente als ein entscheidendes Kriterium für eine genaue Knochenreferenzierung anzusehen ist. Somit kann mittels eines patientenspezifischen Instruments eine genaue Umsetzung der präoperativen Planung erfolgen.

Das grundsätzliche Konzept von patientenspezifischen Instrumenten zur Referenzierung von Knochenstrukturen wird in der Offenlegungsschrift DE 4219939 A1 [K. Radermacher] beschrieben. Patientenspezifische Instrumente werden in der Literatur auch als Schablonen bezeichnet.

In der einfachsten Form werden die patientenspezifischen Instrumente direkt zur Schnitt-und/oder Bohrführung verwendet, indem an den geplanten Stellen, an welchen ein Schnitt oder eine Bohrung am Knochen angebracht werden soll, entsprechende Führungen am patientenspezifischen Instrument für das Schneidwerkzeug oder Bohrwerkzeug angebracht sind. Es ist aber auch möglich, die patientenspezifischen Instrumente nur zur Referenzierung der Anatomie für computer-, oder robotergestützte Eingriffe zu verwenden. In dieser Ausführung können die patientenspezifischen Instrumente keine Schnittführung beinhalten, sondern dienen der Referenzierung der Anatomie (z.B. Anbringen eines optischen Markers am patientenspezifischen Instrument und Messen dieser Position mittels eines Positionserfassungsgeräts, beispielsweise einer Kamera.

In weiteren Dokumenten, beispielsweise der US 20090088763 A1 [L. Aram], US 20140066720 A1, [Smith & Nephew], US 8608748 B2 [Biomet], US 20120239045 A1 [Zimmer Inc.], US 8641721 B2 [DePuy Synthes] werden verschiedene Anwendungen und Ausprägungen von Schablonen und Schnittblöcken beschrieben. Insbesondere für die Kniegelenksendoprothetik existiert eine Vielzahl von verschiedenen Implementierungen. Diese unterscheiden sich hinsichtlich ihrer Aufsetzstrategie und dem Anwendungsgebiet. Auch die Ausprägung der Aufsetzflächen ist unterschiedlich, abhängig von der Anatomie und der verwendeten Bildgebung. Grundsätzlich ist ein möglichst grosser Aufsetzbereich gewünscht, um eine stabile Positionierung und Orientierung zu ermöglichen. Da aber der Knochen und/oder Knorpel nicht an allen Stellen genau rekonstruiert werden kann und intraoperativ Einschränkungen bezüglich der Zugänglichkeit gegeben sind, muss der Aufsetzbereich eingeschränkt oder in einzelne Unterbereiche unterteilt werden. Es gibt Implementierungen, welche mit einer Fläche kontinuierlich auf die bestehende Anatomie aufsetzen, und solche, welche nur auf kleine Teilbereiche aufsetzen. Zudem werden verschiedene Ausprägungen der Kontaktfläche beschrieben, siehe WO 2011156755 A1 [Smith and Nephew].

WO2014/026083 A1) zeigt in Fig. 19 eine Markierungsstelle, die als Kreuzungspunkt zweier balkenförmiger Verbindungselemente ausgebildet ist. Diese Markierungsstelle kann zur visuellen Überprüfung der korrekten Positionierung eines patientenspezifischen Instruments verwendet werden.

WO 2012152900 A1 [Materialize Inc.] beschreibt die Möglichkeit zur Kontrolle über eine oder mehrere Landmarken mittels Ausrichthilfen. Diese Ausrichthilfen bieten dem Operateur eine Möglichkeit zur Kontrolle mittels anatomischer Landmarken. Die beschriebenen Methoden beinhalten keine Vereinfachung beim Aufsetzen des Schnittblockes.

Die nach Stand der Technik beschriebenen Schnittblöcke sind so entwickelt, dass sie eine genaue Positionierung des Schnittblockes auf dem Knochen erlauben. Es ist aber intraoperativ für den Arzt schwierig, diese eindeutige Position aufzufinden. In vielen Fällen gibt es mehrere Stellungen, die eine stabile Position des Schnittblockes vorgeben, aber nicht der geplanten Endposition entsprechen. Zudem bieten die Schnittblöcke wenig Kontrollmöglichkeiten, um die korrekte Positionierung optisch oder haptisch zu überprüfen, da sie auf dem Knochen unmittelbar aufliegen, d.h. den Knochen berühren müssen.

Gerade bei der Anwendung im Kniegelenksersatz sind schon kleine Winkel- und Positionierungsfehler entscheidend für eine erfolgreiche Implantation und Langlebigkeit des künstlichen Gelenkes. Gemäss der Literatur ist eine der häufigsten Komplikationen beim Einsatz von patientenspezifischen Schnittblöcken, dass ein eindeutiges Aufsetzen des Schnittblocks nicht möglich ist. In diesen Fällen muss die Operation konventionell ausgeführt werden oder es muss mit grösseren Fehlern in der Positionierung der Implantatkomponenten gerechnet werden.

Zwar wird in der WO2012152900 vorgeschlagen, ein Ausrichtelement zu verwenden, welches dem Arzt ermöglicht, zu prüfen, ob sich das patientenspezifische Instrument in der aus einem medizinischen Bildgebungsverfahren vorbekannten und festgelegten Position der Knochenoberfläche befindet. Bei Übereinstimmung des Ausrichtelements mit der vorbekannten und vorgegebenen Position unter Einhaltung einer vorgegebenen Blickrichtung ist sichergestellt, dass sich das patientenspezifische Instrument in der korrekten Position auf der Knochenoberfläche befindet.

Aufgabe der vorliegenden Erfindung ist es, ein patientenspezifisches Instrument dahingehend weiterzuentwickeln, dass ein intraoperativ eineindeutiges und reproduzierbares Aufsetzen des patientenspezifischen Instrumentes während des operativen Eingriffs ermöglicht wird und eine optische und/oder haptische Kontrolle der korrekten Positionierung während des operativen Eingriffs ermöglicht wird.

Das korrekte und sichere Aufsetzen des patientenspezifischen Instrumentes in der intraoperativen Anwendung, das heisst während des operativen Eingriffs ist eines der Hauptprobleme für die Anwendung dieser Technologie. Die vorliegende Erfindung macht den Einsatz von patientenspezifischen Instrumenten zuverlässiger und sicherer.

Die Aufgabe wird durch die Merkmale der unabhängigen Patentansprüche gelöst. Vorteilhafte Weiterbildungen ergeben sich aus den abhängigen Patentansprüchen. Der Vorteil der Erfindung besteht darin, dass patientenspezifische Instrumente einfacher intraoperativ aufgesetzt werden können, da deren Grobposition durch eine Ausrichtstruktur vorgegeben ist. Zudem ist eine optische Kontrolle der korrekten Aufsetzposition möglich, was das Risiko einer Fehlpositionierung und damit einer Fehlstellung des patientenspezifischen Instruments in der Operation stark vermindert. Die beschriebenen Ausführungsvarianten sind je nach zu behandelndem Körperteil, das heisst, der während des operativen Eingriffs zugänglichen Anatomie, insbesondere der Knochenstruktur, abhängig vom operativen Zugang und dem geplanten Eingriff angebracht.

Ein patientenspezifisches Instrument umfasst eine oder mehrere Kontaktflächen, die nachfolgend als Aufsetzflächen bezeichnet werden. Insbesondere kann das patientenspezifische Instrument einen Schnittblock enthalten. Diese Aufsetzflächen sind passend zu dem Körperteil, insbesondere der zu referenzierenden Oberfläche des Körperteils, das heisst insbesondere der Knochenoberfläche, ausgebildet. Eine Ausrichtstruktur ist für die Positionierung des patientenspezifischen Instruments vorgesehen. Diese Ausrichtstruktur ermöglicht insbesondere ein einfacheres und zuverlässigeres Aufsetzen des patientenspezifischen Instrumentes auf dem Körperteil, insbesondere auf der Oberfläche des Körperteils. Die Ausrichtstruktur weist einen Abstand zur Oberfläche des Körperteils auf. Die Ausrichtstruktur wird beim Aufsetzen entlang der Oberfläche des Körperteils geführt, mit anderen Worten entlang verschiedener anatomischer Merkmale der Knochenoberfläche geführt. Dieser Abstand zwischen der Ausrichtstruktur und dem Körperteil kann konstant oder variabel sein. Dieser Abstand kann mindestens 0,1 mm betragen. Der Abstand kann in einem Bereich von mindestens 0,1 mm bis maximal 5 mm liegen. Insbesondere kann der Abstand in einem Bereich von mindestens 0,5 mm bis maximal 5 mm liegen.

Zudem sind verschiedene Ausführungen dieser Ausrichtstruktur möglich, welche je nach Anwendung fest, flexibel, schwenk- und/oder abnehmbar mit der Aufsetzfläche verbunden sein können.

Die Ausrichtstruktur ermöglicht insbesondere ein vereinfachtes und eindeutiges Aufsetzen des Instrumentes und/oder ermöglicht eine optische Kontrolle der korrekten Aufsetzposition und/oder ermöglicht eine haptische Kontrolle der korrekten Aufsetzposition.

Nach einem Ausführungsbeispiel kann die Ausrichtstruktur eine Offset-Oberfläche ausbilden oder enthalten, die in einem konstanten Abstand zum Körperteil oder in einem variablen Abstand zum Körperteil angeordnet sein kann. Beispielsweise kann die Ausrichtstruktur eine Aufsetzfläche aufweisen, sowie mindestens ein Armelement, welches in einem konstanten oder variablen Abstand zum Körperteil angeordnet ist.

Die Ausrichtstruktur kann nach einem Ausführungsbeispiel entlang prominenter Kanten entlang der Oberfläche des Körperteils geführt werden oder zu prominenten Landmarken, beispielsweise Erhebungen, Senkungen der Oberfläche des Körperteils geführt werden oder entlang von Gewebegrenzen geführt werden oder zu einem oder mehreren Osteophyten geführt werden oder zu Ansätzen von Sehnen- Bandstrukturen und/oder anderen Weichteilstrukturen geführt werden. Selbstverständlich kann hierfür jedes der genannten Charakteristika der Oberfläche des Körperteils einzeln oder in einer beliebigen Kombination dazu verwendet werden, um die Ausrichtung des patientenspezifischen Instruments anhand der Charakteristika oder Merkmale der Oberfläche des Körpers zu ermöglichen.

Gemäss eines Ausführungsbeispiels enthält die Ausrichtstruktur ein Armelement, welches sich von der Aufsetzfläche in Richtung eines optisch erkennbaren Charakteristikums der Oberfläche des Körperteils erstreckt. Ein derartiges Armelement der Ausrichtstruktur ist daher als Führungsarm ausgestaltet. Das Armelement kann sich von einem anderen Armelement in seinem Querschnitt und seiner Längsabmessung unterscheiden. Jedes der Armelemente kann auch einen in Richtung der Längsabmessung sich verändernden Querschnitt aufweisen. Beispielsweise kann jedes der Armelemente eine oder mehrere Verdickungen, vorzugsweise als Rippen ausgeführt, enthalten, oder ein oder mehrere Verdickungen aufweisen, welche über definierten anatomischen Landmarken angebracht sind, wenn das patientenspezifische Instrument korrekt positioniert ist. Nach einem Ausführungsbeispiel kann das Armelement eine Längsachse aufweisen, die als Spline-Kurve entlang der Oberfläche des Körperteils verläuft. Diese Spline-Kurve kann durch mehrere Stützpunkte oder Landmarken verlaufen und/oder durch diese Stützpunkte oder Landmarken definiert werden.

Gemäss eines Ausführungsbeispiels ist das Armelement als formstabiles Element ausgebildet. Das heisst, die räumliche Anordnung des Armelements in Bezug auf die Ausrichtstruktur ist vorgegeben. Da die Ausrichtstruktur nicht auf dem Körperteil aufliegen soll, ist ein Aufliegen des Armelements auf der Oberfläche des Körperteils nicht nötig und nicht erwünscht, da das Aufliegen dazu führen könnte, dass das Armelement und damit die Ausrichtstruktur in einer fehlerhaften Position am Körperteil angeordnet werden könnten, insbesondere wenn das Armelement beliebig an die Oberfläche des Körperteils angepasst werden könnte, d.h. plastisch verformbar wäre.

Wird ein als formstabiles Element ausgebildetes Armelement in einem bestimmten Abstand zum Körperteil angeordnet, ist die optische Überprüfung von dessen Position auf der Oberfläche des Körperteils erleichtert. Eine fehlerhafte Position kann in vielen Fällen auch dadurch festgestellt werden, indem der Körperteil in den Weg kommt, das heisst, ein Widerstand spürbar ist, wenn die Position des Armelements fehlerhaft ist. Das Armelement ist daher in dieser fehlerhaften Position inkompatibel zum Körperteil. Diese Inkompatibilität ist fühlbar, daher ist eine haptische Kontrolle der Position der Ausrichtstruktur möglich, weil das Armelement beispielsweise unerwartet auf einen Widerstand auf der Körperoberfläche trifft.

Nach der Erfindung enthält die Ausrichtstruktur ein flexibles Material , welches unter Anwendung einer Druckkraft in seiner Form veränderbar ist. Die Druckkraft kann mindestens 10 N betragen. Wenn auf die Ausrichtstruktur bewusst eine Druckkraft aufgebracht wird, wird sie unter Druck nachgiebig.

Das heisst, unter Anwendung einer Druckkraft wird die Ausrichtstruktur flexibel, sodass ihre Form der

Ausrichtstruktur unter dieser Bedingung geändert werden kann. Diese Formänderung erfolgt als Folge der Anwendung der Druckkraft immer bewusst durch den Operateur, das heisst, dass der Operateur immer eine bewusste Handlung vornehmen muss, wenn er die Form der Ausrichtstruktur, insbesondere die Form des Armelements, verändert. Das bedeutet, dass der Operateur die Ausrichtstruktur willentlich und bewusst anpassen kann. Beispielsweise kann er während des Eingriffs feststellen, dass das patientenspezifische Instrument anders positioniert werden muss, als es in der präoperativen Planung vorgesehen gewesen ist, wenn beispielsweise das Design des patientenspezifischen Instruments auf einer Modellierung basiert, die während der präoperativen Planung des Eingriffs erfolgt ist.

Eine im Sinn der vorhergehenden Ausführung flexible Ausrichtstruktur kann beispielsweise durch sehr dünne Armelemente erreicht werden. Ein unter Druckbelastung flexibles Material kann beispielsweise Silikon oder ein Kunststoff, insbesondere ein Polyamid umfassen. Insbesondere kann es sich um einen Kunststoff handeln, der als Rohstoff in einem additiven Herstellungsverfahren verwendet werden kann.

Mittels eines additiven Herstellungsverfahrens kann die Ausrichtstruktur in beliebiger Wandstärke, Länge, mit einer beliebigen Anzahl an Verzweigungen, beispielsweise als Gitterstruktur oder enthaltend eine Gitterstruktur, hergestellt werden.

Nach einem Ausführungsbeispiel können eine oder mehrere Ausrichtstrukturen als Oberflächenstruktur gestaltet sein, welche entlang der der Oberfläche des Körperteils geführt werden. Insbesondere kann die Oberflächenstruktur aus flexiblem Material gestaltet sein oder ein flexibles Material enthalten oder die Oberflächenstruktur kann als Gitterstruktur ausgestaltet sein oder eine Gitterstruktur enthalten.

Nach einem Ausführungsbeispiel können eine oder mehrere Ausrichtstrukturen fest mit dem patientenspezifischen Instrument verbunden sein.

Nach einem Ausführungsbeispiel können eine oder mehrere Ausrichtstrukturen in einer definierten Position am patientenspezifischen Instrument angebracht sein. Unter einer definierten Position wird hierbei eine Position verstanden, in welcher die Ausrichtstruktur und der Schnittblock zueinander durch eindeutig beispielsweise durch ein dreidimensionales, kartesisches Koordinatensystem definierte Punktsysteme festgelegt sind. Jeder Punkt des Punktsystems des Schnittblocks ist eindeutig jedem Punkt der Ausrichtstruktur zugeordnet, sodass der Abstand zwischen jedem beliebigen Punkt des Schnittblocks zu je einem Punkt der Ausrichtstruktur durch einen in Länge und Winkel definierten Richtungsvektor abgebildet werden kann.

Nach einem Ausführungsbeispiel kann die Ausrichtstruktur vom Instrument zumindest teilweise entfernbar sein. Insbesondere kann die Ausrichtstruktur zumindest ein Element der Gruppe der Klappmechanismen, Schwenkmechanismen, Handgriffe, Werkzeuge enthalten.

Beispielsweise kann über einen Klapp- oder Schwenkmechanismus die Ausrichtstruktur in die vorgesehene Position gebracht werden kann, oder die Ausrichtstruktur kann derart angebracht sein, dass sie von Hand oder mit einem Werkzeug weggebogen, respektive ganz oder teilweise entfernt werden kann.

Wenn der Begriff "beispielsweise" in dieser Beschreibung verwendet wird, bezieht sich dieser Begriff auf Ausführungsbeispiele und/oder Ausführungsformen, was nicht notwendigerweise als eine bevorzugtere Anwendung der Lehre der Erfindung zu verstehen ist. In ähnlicher Weise sind die Begriffe "vorzugsweise", "bevorzugt" zu verstehen, indem sie sich auf ein Beispiel aus einer Menge von Ausführungsbeispielen und/oder Ausführungsformen beziehen, was nicht notwendigerweise als eine bevorzugte Anwendung der Lehre der Erfindung zu verstehen ist. Dementsprechend können sich die Begriffe "beispielsweise", "vorzugsweise" oder "bevorzugt" auf eine Mehrzahl von Ausführungsbeispielen und/oder Ausführungsformen beziehen.

Nach einem Ausführungsbeispiel kann das patientenspezifische Instrument ein erstes Teilelement und ein zweites Teilelement umfassen, wobei das erste Teilelement vom zweiten Teilelement abkoppelbar ist. Beispielsweise ist das erste Teilelement ist vollständig entfernbar, da es für die spätere Operation beispielsweise den Schnitt oder die Bohrung nicht erforderlich ist, sondern nur beim Aufsetzen des patientenspezifischen Instruments auf dem Körperteil benötigt wird. Ein wesentlicher Vorteil dieses Ausführungsbeispiels ist, dass nach Entfernen des ersten Teilelements bei der Operation bessere Einsicht auf den zu behandelnden Körperteil gegeben ist. Auch kann das patientenspezifische Instrument an Strukturen des Körperteils ansetzen, welche später weggesägt werden. Das zweite Teilelement enthält einen Schnittblock oder ein Führungselement für ein Bohrwerkzeug. Alternativ kann das erste Teilelement einen Schnittblock oder ein Führungselement für ein Bohrwerkzeug enthalten. Gemäss dieser Variante ist das zweite Teilelement vom ersten Teilelement abkoppelbar.

Ein Verfahren, das nicht Teil der beanspruchten Erfindung ist, zur Positionierung eines patientenspezifischen Instruments umfasst eine oder mehrere Aufsetzflächen, welche passend zu dem Körperteil ausgebildet sind, wobei der Körperteil eine Oberfläche aufweist, wobei das patientenspezifische Instrument mittels einer Ausrichtstruktur auf dem Körperteil positioniert wird, indem die Ausrichtstruktur während der Positionierung in einem Abstand zur Oberfläche des Körperteils geführt wird. Insbesondere kann das patientenspezifische Instrument einen Schnittblock umfassen.

Die Ausrichtstruktur kann entlang prominenter Kanten entlang der Oberfläche des Körperteils geführt werden, bis die Ausrichtstruktur über die prominenten Kanten zu liegen kommt oder zu prominenten anatomischen Landmarken, beispielsweise Erhebungen, Senkungen geführt wird, bis die Ausrichtstruktur über die prominenten Landmarken zu liegen kommt oder eine andere Zielposition in Bezug auf die Landmarke einnimmt, oder entlang von Gewebegrenzen geführt wird, bis die Ausrichtstruktur über die Gewebegrenzen zu liegen kommt oder eine andere Zielposition in Bezug auf die Gewebegrenzen einnimmt oder zu einem oder entlang mehrerer Osteophyten geführt wird oder eine andere Zielposition in Bezug auf die Osteophyten einnimmt, oder zu Ansätzen von Sehnen oder Bandstrukturen oder anderen Weichteilstrukturen geführt wird, bis die Ausrichtstruktur über die Sehnen oder Bandstrukturen oder anderen Weichteilstrukturen zu liegen kommt oder eine andere Zielposition in Bezug auf die Sehnenstrukturen oder Bandstrukturen oder anderen Weichteilstrukturen einnimmt.

Die nachfolgende detaillierte Beschreibung enthält verschiedene Ausführungsbeispiele für das erfindungsgemässe patientenspezifische Instrument. Das patientenspezifische Instrument kann mittels unterschiedlicher Ausrichtstrukturen ausgeführt werden, sodass die Beschreibung einer bestimmten Ausrichtstruktur nur als beispielhaft anzusehen ist. In der Beschreibung und den Ansprüchen werden die Begriffe "enthalten", "umfassen", "aufweisen" als "enthalten, aber nicht beschränkt auf.." interpretiert.

Nachfolgend wird das erfindungsgemässe patientenspezifische Instrument anhand einiger Ausführungsbeispiele dargestellt. Es zeigen
- Fig. 1a und Fig. 1b:: ein Knochenmodell sowie ein patientenspezifisches Instrument ohne Ausrichtstrukturen gemäss des Standes der Technik,
- Fig. 2a und Fig. 2b:: ein Knochenmodell sowie ein patientenspezifisches Instrument mit Ausrichtstrukturen nach einem ersten Ausführungsbeispiel,
- Fig. 3a und Fig. 3b:: ein Knochenmodell sowie ein patientenspezifisches Instrument mit Ausrichtstrukturen nach einem zweiten Ausführungsbeispiel,
- Fig. 4a:: eine Detailansicht von Fig. 3b enthaltend eine Ausrichtstruktur gemäss einer ersten Variante,
- Fig. 4b:: eine Detailansicht von Fig. 3b enthaltend eine Ansicht der Ausrichtstruktur gemäss Fig. 4a,
- Fig. 5:: ein patientenspezifisches Instrument enthaltend eine Ausrichtstruktur gemäss einer zweiten Variante,
- Fig. 6:: ein patientenspezifisches Instrument enthaltend eine Ausrichtstruktur gemäss einer dritten Variante,
- Fig. 7:: ein patientenspezifisches Instrument enthaltend eine Ausrichtstruktur gemäss einer vierten Variante, welche vom patientenspezifischen Instrument abgekoppelt werden kann,
- Fig. 8:: ein patientenspezifisches Instrument enthaltend eine Ausrichtstruktur gemäss einer fünften Variante, welche relativ zum patientenspezifischen Instrument drehbar ist,
- Fig. 9:: ein patientenspezifisches Instrument enthaltend eine Ausrichtstruktur gemäss einer sechsten Variante, welche vom patientenspezifischen Instrument abgekoppelt werden kann.

Fig. 1a zeigt ein Körperteil 10, in diesem Beispiel eine Abbildung eines Femur-Knochens.

Fig. 1b zeigt ein darauf passendes patientenspezifisches Instrument 100, umfassend einen Schnittblock 102 mit vier Aufsetzflächen 104, 106, 108, 110. Dieses patientenspezifische Instrument 100 ist intraoperativ schwierig aufzusetzen, da die vier Stützstellen nicht einfach auffindbar sind und die korrekte Orientierung des patientenspezifischen Instruments 100 optisch nicht direkt ersichtlich ist. Zudem lassen sich auch falsche Aufsetzpositionen finden, bei welchem die vier Stützstellen auf dem Körperteil aufliegen.

Fig. 2a zeigt denselben Körperteil 10 wie Fig. 1a. Der Körperteil 10 weist einen Körperteilrand 11 auf, der für die Ausrichtung eines patientenspezifischen Instruments verwendet werden kann. Für das dargestellte Femurgelenk ist der Gelenkflächenrand mit einer strichlierten Linie markiert.

Fig. 2b zeigt ein patientenspezifisches Instrument 1 in einer in dieser Erfindung beschriebenen Ausführung. Das patientenspezifische Instrument 1 umfasst einen Schnittblock 2 mit vier Aufsetzflächen 4, 6, 8, 12, welche vier Stützstellen für das patientenspezifische Instrument 1 darstellen. Die zusätzlich angebrachten Ausrichtstrukturen 14, 16, 18 geben die grobe Orientierung des Körperteils 10 vor und führen den Körperteil 10 in die richtige Position. Insbesondere kann es sich bei dem Körperteil 10 um einen Knochen, beispielsweise einen Knochen des Kniegelenks handeln. Auch optisch ist für den Operateur einfacher ersichtlich, wie das patientenspezifische Instrument 1 anzubringen ist. Die Ausrichtstrukturen 14, 16, 18 folgen den sogenannten Landmarken des Körperteils 10, also Partien, die mit freiem Auge besonders gut sichtbar sind und sich daher als markante Punkte oder Linien für die Positionierung der Ausrichtstrukturen 14, 16, 18 eignen. Durch die Ausrichtstrukturen 14, 16, 18 kann somit durch visuelle Überprüfung am Einsatzort, das heisst, am Körperteil 10 selbst, eine falsche Positionierung vermieden werden und die vier Stützstellen werden an den vorgesehenen Stellen am Körperteil 10 platziert.

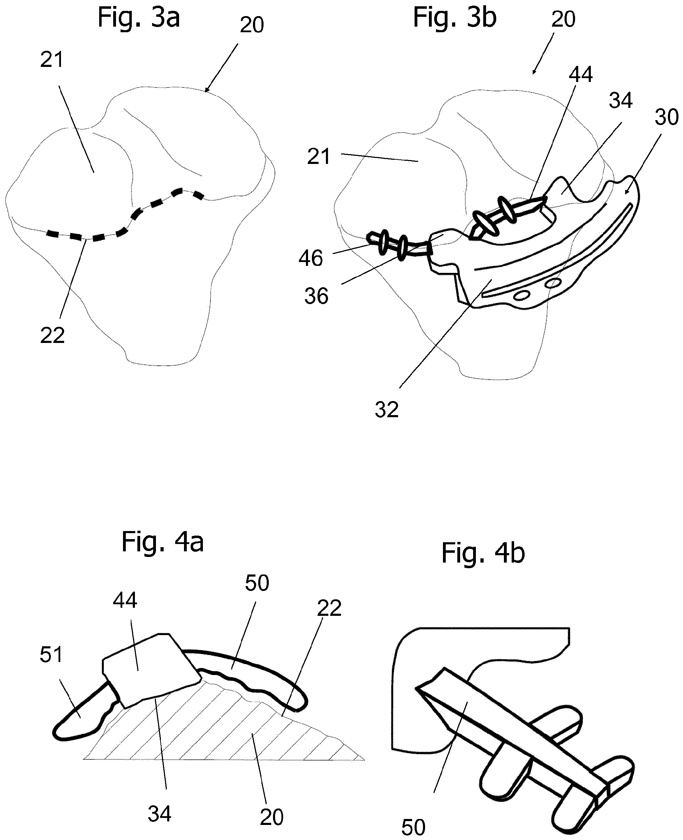

Fig. 3a zeigt einen Körperteil 20, in diesem Beispiel einen Tibia-Knochen, und Fig. 3b zeigt ein patientenspezifisches Instrument 30 für diesen Körperteil 20. Der Tibia-Knochen weist ein Tibiaplateau 21 auf, welches einen Rand 22 aufweist. Der Rand 22 des Tibiaplateaus ist in Fig. 3a teilweise mit einer strichlierten Linie gekennzeichnet. Dieser Rand 22 ist ein Ausführungsbeispiel für eine Landmarke des Körperteils 20. Das patientenspezifische Instrument 30 umfasst einen Schnittblock 32 mit zumindest zwei Aufsetzflächen 34, 36, welche zwei Stützstellen für das patientenspezifische Instrument 30 darstellen. Die zusätzlich angebrachten Ausrichtstrukturen 44, 46 geben die grobe Orientierung des patientenspezifischen Instruments 30 vor, welches auf dem Körperteil 20 platziert werden soll. Mittels der Ausrichtstrukturen 44, 46 kann das patientenspezifische Instrument in Bezug auf den Körperteil 20 in die richtige Position gebracht werden. Die Ausrichtstrukturen 44, 46 berühren dabei den Körperteil 20 nicht, sie sind in einem Abstand zum Körperteil 30 angeordnet. Auch optisch ist für den Operateur einfacher ersichtlich, wie das patientenspezifische Instrument 30 anzubringen ist. Die Ausrichtstrukturen 44, 46 folgen dem Verlauf der Landmarken auf dem Körperteil 20. Durch die Ausrichtstrukturen 44, 46 kann eine falsche Positionierung des patientenspezifischen Instruments vermieden werden und die zwei Stützstellen werden an den vorgesehenen Stellen am Körperteil platziert. Die Ausrichtstrukturen 44, 46 sind gemäss dieses Ausführungsbeispiels als Armelemente ausgebildet, die sich von der Ausrichtstruktur in verschiedene Richtungen erstrecken. Die Armelemente können insbesondere entlang des Randes 22 des Tibiaplateaus 21 angeordnet werden, ermöglichen eine höhere Aufsetzgenauigkeit, sowie einen präzise kontrollierbaren Aufsetzvorgang. Ohne die Verwendung der Armelemente ist eine ungewollte Verkippung des patientenspezifischen Instruments 30 nur schwer feststellbar.

Fig. 4a zeigt eine Detailansicht einer Ausrichtstruktur, beispielsweise der Ausrichtstruktur 44 der Fig. 3b, welche einen Schnitt durch die Armelemente zeigt. Die Ausrichtstruktur 44 enthält die Armelemente 50, 51, die im vorliegenden Ausführungsbeispiel nicht dem genauen Aufsetzen des patientenspezifischen Instruments 30 auf den Körperteil 20. Daher sind die Armelemente 50, 51 leicht abgesetzt vom Körperteil 20 platziert (im Beispiel 1 mm) oder flexibel konstruiert. Fig. 4a zeigt somit den Abstand der Armelemente 50, 51 von der Oberfläche des Körperteils 20. Der Körperteil 20 liegt auf der vorgesehenen Kontaktfläche, welche durch die Aufsetzfläche 34 gebildet ist, auf.

Nach einem nicht dargestellten Ausführungsbeispiel laufen die Ausrichtstrukturen entlang von Osteophyten an der Knochen-/Knorpelgrenze. Gemäss dieses Ausführungsbeispiels erstrecken sich zwei Armelemente 50, 51 von der Ausrichtstruktur 44 in zwei unterschiedliche Richtungen. Die Richtung ist gemäss dieses Ausführungsbeispiels durch den Rand 22 des Tibiaplateaus 20 vorgegeben.

Fig. 4b zeigt eine Ansicht eines Armelements 50 der Ausrichtstruktur der Fig. 4a. Diese Ausrichtstruktur kann auch für jede der vorhergehenden oder in der Folge beschriebenen Ausführungsbeispiele zum Einsatz gelangen. Das Armelement weist eine Mehrzahl von Fingerelementen auf.

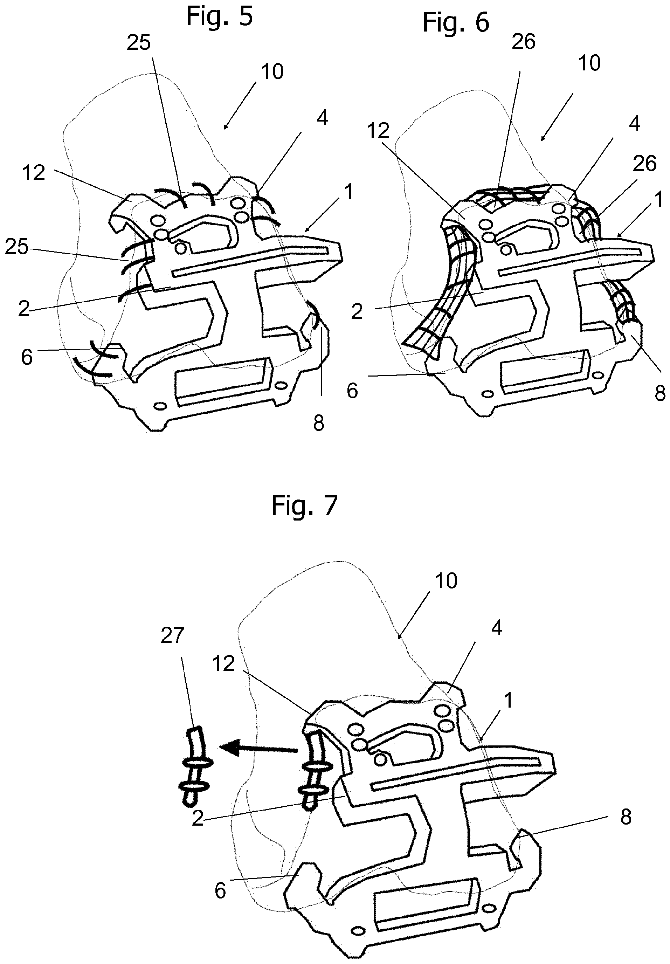

Fig. 5 zeigt ein patientenspezifisches Instrument 1 gemäss Fig. 2b enthaltend eine Variante einer Ausrichtstruktur, wobei eine derartige Ausrichtstruktur in ähnlicher Weise auch für ein patientenspezifisches Instrument 30 gemäss Fig. 3b zum Einsatz kommen könnte. Das patientenspezifische Instrument 1 umfasst einen Schnittblock 2 mit vier Aufsetzflächen 4, 6, 8, 12, welche vier Stützstellen für das patientenspezifische Instrument 1 darstellen. Die zusätzlich angebrachte Ausrichtstruktur 25 gibt die grobe Orientierung des Körperteils 10 vor und führt den Körperteil 10 in die richtige Position. Auch optisch ist durch die Ausrichtstruktur 25 für den Operateur einfacher ersichtlich, wie das patientenspezifische Instrument 1 anzubringen ist. Durch die Ausrichtstruktur 25 kann eine falsche Positionierung des patientenspezifischen Instruments 1 vermieden werden und die vier Stützstellen werden an den vorgesehenen Stellen am Körperteil 10 platziert. Die Ausrichtstruktur 25 ist aus mehreren dünnen Armelementen gebildet, welche entlang definierter Oberflächenstrukturen (hier Osteophyten entlang Knorpel-/Knochengrenze) geführt werden. Die Armelemente können verschiedenartig ausgebildet sein, um eine möglichst eindeutige Positionierung zu gewährleisten. Die Armelemente können flexibel ausgestaltet sein, um Unterschiede zwischen einem 3D-Knochenmodell und dem Körperteil auszugleichen. Das 3D-Knochenmodell wurde zur Herstellung der Ausrichtstruktur verwendet. Die Anordnung der Ausrichtstruktur erfolgt anhand der Vorgaben des 3D-Körperteilmodells. Da das 3D Körperteilmodell aus Patientendaten erzeugt werden kann, kann mittels dieses patientenspezifischen 3D-Körperteilmodells ein auf den zu behandelnden Körperteil des Patienten abgestimmtes patientenspezifisches Instrument erzeugt werden. Beispielsweise kann ein derartiges patientenspezifisches Instrument mittels eines additiven Herstellungsverfahrens hergestellt werden.

Fig. 6 zeigt ein patientenspezifisches Instrument 1 gemäss Fig. 2b enthaltend eine Variante einer Ausrichtstruktur, wobei eine derartige Ausrichtstruktur in ähnlicher Weise auch für ein patientenspezifisches Instrument 30 gemäss Fig. 3b zum Einsatz kommen könnte. Das patientenspezifische Instrument 1 umfasst einen Schnittblock 2 mit vier Aufsetzflächen 4, 6, 8,12, welche vier Stützstellen für das patientenspezifische Instrument 1 darstellen. Die zusätzlich angebrachte Ausrichtstruktur 26 gibt die grobe Orientierung des patientenspezifischen Instruments 1 vor und führt das patientenspezifische Instrument 1 am Körperteil 10 in die richtige Position.

Durch die Ausrichtstruktur 26 kann eine falsche Positionierung des patientenspezifischen Instruments 1 vermieden werden und die vier Stützstellen werden an den vorgesehenen Stellen am Knochen platziert. Die Ausrichtstruktur 26 ist aus mehreren dünnen Armelementen gebildet, die ein Oberflächenelement aufspannen, das entlang der Knochenoberfläche in einem kleinen Abstand ausgebildet ist, so dass das Oberflächenelement den Knochen nicht berührt und das patientenspezifische Instrument 1 nur auf den vorgesehenen Aufsetzflächen 4, 6, 8, 12 aufliegt. Das Oberflächenelement kann verschiedenartig ausgebildet sein, z.B als Gitter-/Wabenstruktur, um den Körperteil 10 nicht zu verdecken. Das Oberflächenelement kann auch ein transparentes Material enthalten. Das Oberflächenelement kann aus einer Mehrzahl flexibler Armelemente ausgeführt sein, um Unterschiede zwischen dem 3D-Körperteilmodell und dem Körperteil auszugleichen.

Fig. 7 zeigt ein patientenspezifisches Instrument 1 gemäss Fig. 2b enthaltend eine Variante einer Ausrichtstruktur, wobei eine derartige Ausrichtstruktur in ähnlicher Weise auch für ein patientenspezifisches Instrument 30 gemäss Fig. 3b zum Einsatz kommen könnte. Das patientenspezifische Instrument 1 umfasst einen Schnittblock 2 mit vier Aufsetzflächen 4, 6, 8, 12, welche vier Stützstellen für das patientenspezifische Instrument 1 darstellen. Die zusätzlich angebrachte Ausrichtstruktur 27 gibt die grobe Orientierung des patientenspezifischen Instruments 1 vor und führt das patientenspezifische Instrument 1 am Körperteil 10 in die richtige Position.

Die Ausrichtstruktur 27 kann vom patientenspezifischen Instrument 1 abgekoppelt werden, um Kollision mit Weichteilen und/oder anderen Instrumenten im weiteren OP-Verlauf zu vermeiden. Sobald das patientenspezifische Instrument 1 in seiner Zielposition fixiert ist, wird die Ausrichtstruktur 27 nicht mehr benötigt und kann entfernt werden, falls diese den weiteren OP-Ablauf stören sollte. In einem weiteren, in den Fig. nicht dargestellten Ausführungsbeispiel ist denkbar, dass die Ausrichtstruktur 27 ganz oder teilweise von Hand oder mit einem Werkzeug, beispielsweise einer Zange, abgetrennt werden kann.

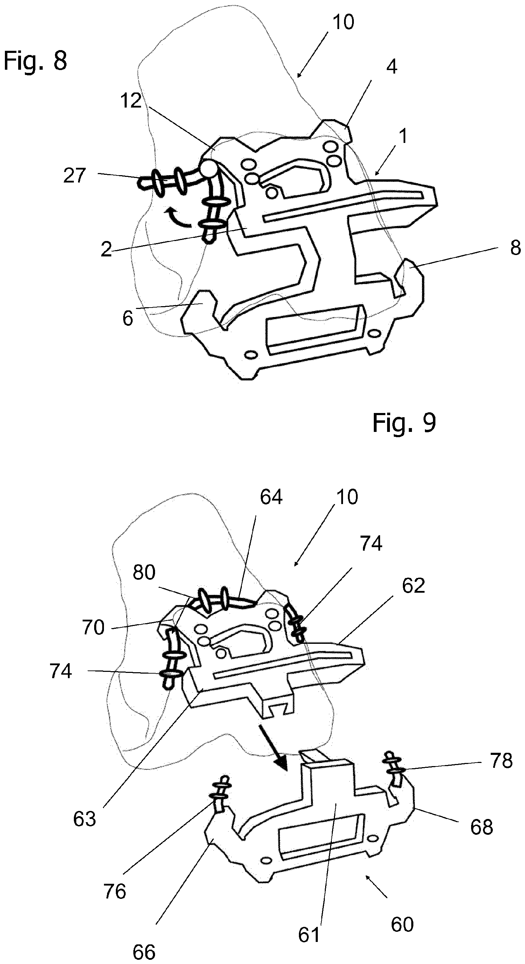

Das Ausführungsbeispiel gemäss Fig. 8 zeigt, dass die Ausrichtstruktur 27 über einen Schwenk-/Klapp- oder anderen Mechanismus bewegt werden kann. Die Ausrichtstruktur 27 kann wie im Ausführungsbeispiel gemäss Fig. 7 vom patientenspezifischen Instrument 1 abgekoppelt werden, um Kollision mit Weichteilen und/oder anderen Instrumenten im weiteren OP-Verlauf zu vermeiden. Sobald das patientenspezifische Instrument 1 in seiner Zielposition fixiert ist, wird die Ausrichtstruktur 27 nicht mehr benötigt und kann in eine andere Position bewegt werden oder entfernt werden, falls diese den weiteren OP-Ablauf stören sollte.

Fig. 9 zeigt ein patientenspezifisches Instrument 60, die ein erstes Teilelement 61 sowie ein zweites Teilelement 63 enthält. Das erste Teilelement 61 ist vollständig vom zweiten Teilelement 63 abtrennbar. Insbesondere kann das erste Teilelement 61 zu einem späteren Zeitpunkt der Operation wieder mit dem zweiten Teilelement 63 verbunden werden. Das erste Teilelement 61 kann mit dem zweiten Teilelement 63 beispielsweise über eine formschlüssige Kopplung verbunden werden. Im vorliegenden Fall enthält das erste Teilelement 61 einen Vorsprung mit trapezförmigem Querschnitt, der in eine passende Schwalbenschwanznut des zweiten Teilelements 63 einführbar ist. Über die formschlüssige Kopplung können auch anders ausgestaltete Teilelemente temporär oder dauerhaft mit dem zweiten Teilelement 63 verbunden werden.

Nur das zweite Teilelement 63 wird auf dem Körperteil 10 fixiert, das heisst insbesondere in definiertem Abstand zum Körperteil 10 positioniert, und später für die Führung des Schneidwerkzeugs für den Knochenschnitt verwendet. Das Teilelement 63 enthält einen Schnittblock 62.

Somit besteht das patientenspezifische Instrument 60 gemäss des Ausführungsbeispiels gemäss Fig. 9 aus dem ersten und zweiten Teilelement. Das erste Teilelement 61 ist vollständig entfernbar, da es für die spätere Operation beispielsweise den Schnitt oder die Bohrung nicht erforderlich ist, sondern nur beim Aufsetzen des patientenspezifischen Instruments 60 auf dem Körperteil benötigt wird. Ein wesentlicher Vorteil dieses Ausführungsbeispiels ist, dass nach Entfernen des ersten Teilelements 61 bei der Operation bessere Einsicht auf den zu behandelnden Körperteil gegeben ist. Auch kann das patientenspezifische Instrument 60 an Strukturen des Körperteils ansetzen, welche später weggesägt werden.

Das patientenspezifische Instrument 60 umfasst einen Schnittblock 62 mit vier Aufsetzflächen 64, 66, 68, 70, welche vier Stützstellen für das patientenspezifische Instrument 1 darstellen. Die Aufsetzflächen 66, 68 befinden sich am ersten Teilelement 61. Die Aufsetzflächen 64, 70 befinden sich am zweiten Teilelement 63, wobei die Aufsetzfläche 70 durch den Schnittblock 62 verdeckt ist. Die zusätzlich angebrachten Ausrichtstrukturen 74, 76, 78, 80 geben die grobe Orientierung des patientenspezifischen Instruments vor und führen das patientenspezifische Instrument 1 in die richtige Position.

Für den Fachmann ist offensichtlich, dass viele weitere Modifikationen zusätzlich zu den beschriebenen Ausführungsbeispielen möglich sind, ohne vom erfinderischen Konzept abzuweichen. Der Gegenstand der Erfindung wird somit durch die vorangehende Beschreibung nicht eingeschränkt und ist durch den Schutzbereich bestimmt, der durch die Ansprüche festgelegt ist. Für die Interpretation der Ansprüche oder der Beschreibung ist die breitest mögliche Lesart der Ansprüche massgeblich. Insbesondere sollen die Begriffe "enthalten" oder "beinhalten" derart interpretiert werden, dass sie sich auf Elemente, Komponenten oder Schritte in einer nicht-ausschliesslichen Bedeutung beziehen, wodurch angedeutet werden soll, dass die Elemente, Komponenten oder Schritte vorhanden sein können oder genutzt werden können, dass sie mit anderen Elementen, Komponenten oder Schritten kombiniert werden können, die nicht explizit erwähnt sind. Wenn die Ansprüche sich auf ein Element oder eine Komponente aus einer Gruppe beziehen, die aus A, B, C... N Elementen oder Komponenten bestehen kann, soll diese Formulierung derart interpretiert werden, dass nur ein einziges Element dieser Gruppe erforderlich ist, und nicht eine Kombination von A und N, B und N oder irgendeiner anderen Kombination von zwei oder mehr Elementen oder Komponenten dieser Gruppe.

## Patentansprüche

1. Ein patientenspezifisches Instrument (1, 30, 60) umfassend eine oder mehrere Aufsetzflächen (4, 6, 8, 12, 34, 36, 64, 66, 68, 70), welche passend zu einem Körperteil (10, 20) ausgebildet sind, wobei der Körperteil (10, 20) als eine Knochenstruktur ausgebildet ist, wobei der Körperteil (10, 20) eine Oberfläche aufweist, wobei das patientenspezifische Instrument (1, 30, 60) eine Ausrichtstruktur (14, 16, 18, 25, 26, 27, 44, 46, 74, 76, 78, 80) für die Positionierung des patientenspezifischen Instrumentes auf dem Körperteil enthält, wobei die Ausrichtstruktur (14, 16, 18, 25, 26, 27, 44, 46, 74, 76, 78, 80) so konfiguriert ist, dass sie einen Abstand zur Oberfläche des Körperteils (10, 20) aufweist, **dadurch gekennzeichnet, dass** die Ausrichtstruktur (14, 16, 18, 25, 26, 27, 44, 46, 74, 76, 78, 80) ein flexibles Material enthält, sodass die Ausrichtstruktur (14, 16, 18, 25, 26, 27, 44, 46, 74, 76, 78, 80) unter Anwendung einer Druckkraft in ihrer Form veränderbar ist, sodass der Operateur die Ausrichtstruktur (14, 16, 18, 25, 26, 27, 44, 46, 74, 76, 78, 80) willentlich und bewusst anpassen kann.

2. Instrument (1, 30, 60) nach Anspruch 1, wobei der Abstand konstant oder variabel ist.

3. Instrument (1, 30, 60) nach einem der vorhergehenden Ansprüche 1 oder 2, wobei die Ausrichtstruktur oder jede der Ausrichtstrukturen (14, 16, 18, 25, 26, 27, 44, 46, 74, 76, 78, 80) fest, flexibel, schwenk- und/oder abnehmbar mit der Aufsetzfläche (4, 6, 8, 12, 34, 36, 64, 66, 68, 70) verbunden ist.

4. Instrument (1, 30, 60) nach einem der vorhergehenden Ansprüche, wobei die Ausrichtstruktur (14, 16, 18, 25, 26, 27, 44, 46, 74, 76, 78, 80) eine Offset-Oberfläche ausbildet, wobei die Offset-Oberfläche in einem konstanten Abstand zum Körperteil oder in einem variablen Abstand zum Körperteil (10, 20) angeordnet ist.

5. Instrument (1, 30, 60) nach einem der vorhergehenden Ansprüche, wobei die Ausrichtstruktur (14, 16, 18, 25, 26, 27, 44, 46, 74, 76, 78, 80) ein Armelement (50, 51) enthält, welches sich von der Aufsetzfläche in Richtung eines optisch erkennbaren Charakteristikums der Oberfläche des Körperteils (10, 20) erstreckt.

6. Instrument (1, 30, 60) nach Anspruch 5, wobei das Armelement (50, 51) einen sich in Richtung der Längsabmessung verändernden Querschnitt aufweist, das Armelement (50, 51) insbesondere eine oder mehrere Verdickungen enthält, vorzugsweise eine Rippe enthält oder das Armelement eine Verdickung aufweist, welche über definierten anatomischen Landmarken angebracht ist, wenn das patientenspezifische Instrument korrekt positioniert ist, welche über mindestens einem der Charakteristika aus der Gruppe der Kanten, der Erhebungen, der Senkungen, der anatomischen Landmarken, der Gewebegrenzen, der Osteophyten, der Ansätze von Sehnenstrukturen oder Bandstrukturen oder Weichteilstrukturen angebracht sein kann oder eine andere Zielposition in Bezug auf die genannten Charakteristika aufweisen kann.

7. Instrument (1, 30, 60) nach einem der Ansprüche 5 oder 6, wobei mindestens eines der Armelemente (50, 51) als formstabiles Element ausgeführt ist.

8. Instrument (1, 30, 60) nach einem der vorhergehenden Ansprüche, wobei die Ausrichtstruktur (14, 16, 18, 25, 26, 27, 44, 46, 74, 76, 78, 80) als Oberflächenstruktur gestaltet ist, welche entlang des Körperteils (10, 20) führbar ist.

9. Instrument (1, 30, 60) nach Anspruch 8, wobei die Oberflächenstruktur eine Gitterstruktur umfasst.

10. Instrument (1, 30, 60) nach einem der vorhergehenden Ansprüche, wobei die Ausrichtstruktur (14, 16, 18, 25, 26, 27, 44, 46, 74, 76, 78, 80) fest mit dem patientenspezifischen Instrument verbunden ist, wobei insbesondere die Ausrichtstruktur in einer definierten Position am patientenspezifischen Instrument angebracht ist.

11. Instrument (1, 30, 60) nach einem der vorhergehenden Ansprüche, wobei die Ausrichtstruktur (14, 16, 18, 25, 26, 27, 44, 46, 74, 76, 78, 80) vom Instrument zumindest teilweise entfernbar ist, wobei die Ausrichtstruktur zumindest ein Element der Gruppe der Klappmechanismen, Schwenkmechanismen, Handgriffe, Werkzeuge enthalten kann.

12. Instrument (60) nach einem der vorhergehenden Ansprüche, umfassend ein erstes Teilelement (61) und ein zweites Teilelement (63), wobei das erste Teilelement (61) vom zweiten Teilelement (63) abkoppelbar ist.

13. Instrument (1, 30, 60) nach einem der vorhergehenden Ansprüche, wobei die Druckkraft mindestens 10 N beträgt.

14. Instrument (1, 30, 60) nach einem der vorhergehenden Ansprüche, wobei die Ausrichtstruktur als eine flexible Ausrichtstruktur ausgebildet ist, wobei die flexible Ausrichtstruktur durch sehr dünne Armelemente erhältlich ist.

15. Instrument (1, 30, 60) nach Anspruch 14, wobei das unter Druckbelastung flexible Material Silikon oder einen Kunststoff, insbesondere ein Polyamid, umfasst.

## Claims

1. A patient-specific instrument (1, 30, 60) comprising one or more contact surfaces (4, 6, 8, 12, 34, 36, 64, 66, 68, 70) which are configured to match a body part (10, 20), wherein the body part (10, 20) is configured as a bone structure, wherein the body part (10, 20) is provided with a surface, wherein the patient-specific instrument (1, 30, 60) comprises an alignment structure (14, 16, 18, 25, 26, 27, 44, 46, 74, 76, 78, 80) for positioning the patient-specific instrument on the body part, wherein the alignment structure (14, 16, 18, 25, 26, 27, 44, 46, 74, 76 , 78, 80) is configured to be spaced from the surface of the body part (10, 20), **characterized in that** the alignment structure (14, 16, 18, 25, 26, 27, 44, 46, 74, 76 , 78, 80) contains a flexible material, such that the alignment structure (14, 16, 18, 25, 26, 27, 44, 46, 74, 76, 78, 80) can be changed in its shape by the application of the compressive force, such that the operator can adjust the alignment structure (14, 16, 18, 25, 26, 27, 44, 46, 74, 76, 78, 80) at will and consciously.

2. The instrument (1, 30, 60) of claim 1, wherein the distance is constant or variable.

3. The instrument (1, 30, 60) of one of claims 1 or 2, wherein the alignment structure or each of the alignment structures (14, 16, 18, 25, 26, 27, 44, 46, 74, 76, 78, 80) is firmly, flexibly, pivotably and/or detachably connected to the contact surface (4, 6, 8, 12, 34, 36, 64, 66, 68, 70).

4. The instrument (1, 30, 60) of one of the preceding claims, wherein the alignment structure (14, 16, 18, 25, 26, 27, 44, 46, 74, 76, 78, 80) forms an offset surface, wherein the offset surface is located at a constant distance from the body part or at a variable distance from the body part (10, 20).

5. The instrument (1, 30, 60) of one of the preceding claims, wherein the alignment structure (14, 16, 18, 25, 26, 27, 44, 46, 74, 76, 78, 80) comprises an arm element (50, 51) which extends from the contact surface in the direction of an optically recognizable characteristic of the surface of the body part (10, 20).

6. The instrument (1, 30, 60) of claim 5, wherein the arm element (50, 51) is provided with a variable cross section in the direction of the longitudinal dimension, wherein the arm element (50, 51) contains in particular one or more thickenings, preferably the arm element contains a rib or has a thickening which is attached over defined anatomical landmarks when the patient-specific instrument is correctly positioned, wherein the patient-specific instrument can be attached over at least one of the characteristics from the group consisting of edges, elevations, depressions, anatomical landmarks, tissue borders, osteophytes, the attachments of tendon structures or ligament structures or soft tissue structures or can have a different target position in relation to the characteristics mentioned.

7. The instrument (1, 30, 60) of one of claims 5 or 6, wherein at least one of the arm elements (50, 51) is designed as a dimensionally stable element.

8. The instrument (1, 30, 60) of one of the preceding claims, wherein the alignment structure (14, 16, 18, 25, 26, 27, 44, 46, 74, 76, 78, 80) is configured as a surface structure which is guidable along the body part (10, 20).

9. The instrument (1, 30, 60) of claim 8, wherein the surface structure comprises a grid structure.

10. The instrument (1, 30, 60) of one of the preceding claims, wherein the alignment structure (14, 16, 18, 25, 26, 27, 44, 46, 74, 76, 78, 80) is firmly connected to the patient-specific instrument, wherein in particular the alignment structure is attached in a defined position at the patient-specific instrument.

11. The instrument (1, 30, 60) of one of the preceding claims, wherein the alignment structure (14, 16, 18, 25, 26, 27, 44, 46, 74, 76, 78, 80) is at least partially removable from the instrument, wherein the alignment structure can contain at least one element from the group consisting of folding mechanisms, pivoting mechanisms, handles, tools.

12. The instrument (60) of one of the preceding claims, further comprising a first portion (61) and a second portion (63), wherein the first portion (61) can be uncoupled from the second portion (63).

13. The instrument (1, 30, 60) of one of the preceding claims, wherein the compressive force amounts to at least 10 N.

14. The instrument (1, 30, 60) of one of the preceding claims, wherein the alignment structure is configured as a flexible alignment structure, wherein the flexible alignment structure is obtainable by very thin arm elements.

15. The instrument (1, 30, 60) of claim 14, wherein the material, which is flexible under pressure, comprises a silicone or a plastic, in particular a polyamide.

## Revendications

1. Un instrument spécifique au patient (1, 30, 60) comprenant une ou plusieurs surfaces de contact (4, 6, 8, 12, 34, 36, 64, 66, 68, 70) qui sont configurées pour correspondre à une partie du corps (10, 20), en ce que la partie du corps (10, 20) est réalisée sous la forme d'une structure osseuse, en ce que la partie du corps (10, 20) présente une surface, en ce que l'instrument spécifique au patient (1, 30, 60) comporte une structure d'alignement (14, 16, 18, 25, 26, 27, 44, 46, 74, 76, 78, 80) pour positionner l'instrument spécifique au patient sur la partie du corps, en ce que la structure d'alignement (14, 16, 18 , 25, 26, 27, 44, 46, 74, 76 , 78, 80) est configurée pour être espacé à une distance de la surface de la partie de corps (10, 20), **caractérisé en ce que** la structure d'alignement (14, 16, 18, 25, 26, 27, 44, 46, 74, 76, 78, 80) contient un matériau flexible, de sorte que la structure d'alignement (14, 16, 18, 25, 26, 27, 44, 46, 74, 76, 78 , 80) peut être modifiée sous l'application de la force de compression, de sorte que l'opérateur peut ajuster la structure d'alignement (14, 16, 18, 25, 26, 27, 44, 46, 74, 76, 78, 80) à volonté et consciemment.

2. Instrument (1, 30, 60) selon la revendication 1, dans lequel la distance est constante ou variable.

3. Instrument (1, 30, 60) selon l'une des revendications 1 ou 2, dans lequel la structure d'alignement ou chacune des structures d'alignement (14, 16, 18, 25, 26, 27, 44, 46, 74, 76, 78, 80 ) est relié de manière fixe, flexible, pivotante et/ou amovible à la surface de contact (4, 6, 8, 12, 34, 36, 64, 66, 68, 70).

4. Instrument (1, 30, 60) selon l'une quelconque des revendications précédentes, dans lequel la structure d'alignement (14, 16, 18, 25, 26, 27, 44, 46, 74, 76, 78, 80) forme une surface décalée, dans laquelle la surface décalée est située à une distance constante de la partie de corps ou à une distance variable de la partie de corps (10, 20).

5. Instrument (1, 30, 60) selon l'une quelconque des revendications précédentes, dans lequel la structure d'alignement (14, 16, 18, 25, 26, 27, 44, 46, 74, 76, 78, 80) comprend un élément de bras (50, 51) qui s'étend depuis la surface de contact dans la direction d'une caractéristique optiquement reconnaissable de la surface de la partie du corps (10, 20).

6. Instrument (1, 30, 60) selon la revendication 5, dans lequel l'élément de bras (50, 51) est pourvu d'une section transversale évolutive dans le sens de la dimension longitudinale, en ce que l'élément de bras (50, 51) contient notamment un ou plusieurs épaississements, et contient de préférence une nervure ou l'élément de bras est pourvu d'un épaississement qui est fixé sur des repères anatomiques définis, lorsque l'instrument spécifique au patient est correctement positionné, qui peut être attaché sur au moins une des caractéristiques du groupe des bords, des élévations, des dépressions, des repères anatomiques, des bordures tissulaires, des ostéophytes, les attaches de structures tendineuses ou de structures ligamentaires ou de structures de tissus mous ou peut avoir une position cible différente par rapport aux caractéristiques mentionnées.

7. Instrument (1, 30, 60) selon l'une des revendications 5 ou 6, dans lequel au moins un des éléments de bras (50, 51) est conçu comme un élément indéformable.

8. Instrument (1, 30, 60) selon l'une quelconque des revendications précédentes, dans lequel la structure d'alignement (14, 16, 18, 25, 26, 27, 44, 46, 74, 76, 78, 80) est conçue en tant que structure de surface qui peut être guidée le long de la partie de corps (10, 20).

9. Instrument (1, 30, 60) selon la revendication 8, dans lequel la structure de surface comprend une structure en grille.

10. Instrument (1, 30, 60) selon l'une quelconque des revendications précédentes, dans lequel la structure d'alignement (14, 16, 18, 25, 26, 27, 44, 46, 74, 76, 78, 80) est fermement connecté à l'instrument spécifique au patient, dans lequel en particulier la structure d'alignement est fixée dans une position définie sur l'instrument spécifique au patient.

11. Instrument (1, 30, 60) selon l'une quelconque des revendications précédentes, dans lequel la structure d'alignement (14, 16, 18, 25, 26, 27, 44, 46, 74, 76, 78, 80) est au moins partiellement amovible de l'instrument 10, en ce que la structure d'alignement peut contenir au moins un élément du groupe des mécanismes de pliage, des mécanismes de pivotement, des poignées, des outils.

12. Instrument (60) selon l'une quelconque des revendications précédentes, que comprend un premier sous-élément (61) et un deuxième sous-élément (63), dans lequel le premier sous-élément (61) peut être désaccouplé du deuxième sous-élément ( 63).

13. Instrument (1, 30, 60) selon l'une quelconque des revendications précédentes, dans lequel la force de compression est d'au moins 10 N.

14. Instrument (1, 30, 60) selon l'une quelconque des revendications précédentes, dans lequel la structure d'alignement est configurée comme une structure d'alignement flexible, en ce que la structure d'alignement flexible peut être obtenue par des éléments de bras très fins.

15. Instrument (1, 30, 60) selon la revendication 14, dans lequel le matériau souple sous pression comprend un silicone ou un plastique, notamment un polyamide.
